# FASCICULE DE BREVET EUROPEEN

(11) **EP 4 629 911 B1**
(45) Date de publication et mention de la délivrance du brevet: **04.11.2026**
(21) Numéro de dépôt: 23818483.2
(22) Date de dépôt: 06.12.2023
(51) Int. Cl.: A61B 17/17, A61B 17/04, A61F 2/08

(54) **INSTRUMENT ANCILLAIRE POUR CHIRURGIE DE LA DISJONCTION ACROMIO-CLAVICULAIRE**
HILFSINSTRUMENT FÜR DIE AKROMIOKLAVIKULARDISJUNKTION-CHIRURGIE
ANCILLARY INSTRUMENT FOR ACROMIOCLAVICULAR DISJUNCTION SURGERY

(30) Priorité: 07.12.2022 FR 2212930
(43) Date de publication de la demande: 15.10.2025
(73) Titulaire: V.I.M.S. Video Interventionnelle Médicale Scientifique, 31620 Villeneuve-lès-Bouloc (FR)
(72) Inventeur: GRAVIER, Renaud, 13008 MARSEILLE (FR); KANY, Jean, 31320 AUREVILLE (FR); ZUMSTEIN, Matthias, 3626 HUENIBACH (CH)
(74) Mandataire: Allen, Caroline Margaret
(86) Numéro de dépôt international: PCT/EP2023/084556
(87) Numéro de publication internationale: WO 2024/121245

(56) Documents cités:
- CN-A- 104 783 873
- KR-B1- 102 035 651
- US-A1- 2012 253 352
- US-B2- 11 331 112

## Description

### Domaine technique de l'invention

L'invention appartient au domaine des instruments chirurgicaux.

L'invention concerne un appareil pour guider une intervention chirurgicale de reconstruction d'un ligament coraco-claviculaire trapézoïde ou une intervention chirurgicale de reconstruction d'un ligament coraco-claviculaire trapézoïde et d'un ligament coraco-claviculaire conoïde.

### Technique antérieure

La disjonction acromio-claviculaire est une luxation de l'articulation acromio-claviculaire qui unit la clavicule à l'acromion. Cette luxation représente 40% des traumatismes de l'épaule d'un athlète. Cette luxation implique généralement, des lésions des ligaments positionnés entre la clavicule et l'apophyse coracoïde. Il s'agit du ligament coraco-claviculaire trapézoïde et du ligament coraco-claviculaire conoïde. A partir d'un certain niveau de gravité des lésions des ligaments coraco-claviculaire, des chirurgies sont déployées mais présentent souvent des complications. Comme l'enseigne notamment le brevet FR 2 692 470, de telles chirurgies, appelées chirurgies de reconstruction de ligament coraco-claviculaire consistent à ajouter un ou plusieurs ligaments artificiels entre la clavicule et l'apophyse coracoïde. Elles impliquent souvent le perçage de tunnels dans la clavicule et parfois dans l'apophyse coracoïde. Afin de faciliter cette chirurgie et de limiter les risques associés, un instrument permettant de guider le perçage est notamment illustré dans le brevet US 9,072,510. Un tel instrument permet d'aider à réaliser une chirurgie de reconstruction du ligament coraco-claviculaire trapézoïde en limitant les risques de complication lors de la chirurgie. En effet, l'utilisation d'un instrument pour cette chirurgie permet notamment de protéger des zones, dites sensibles, denses en nerfs et en artères à l'aide d'une pièce insérée temporairement dans le corps du patient lors de l'opération et positionnée dans un axe selon lequel le perçage est réalisé. Cette pièce permet de bloquer un foret utilisé pour percer, avant qu'il n'atteigne une zone sensible, lors du perçage.

La reconstruction du ligament coraco-claviculaire trapézoïde présente cependant l'inconvénient d'être peu anatomique. En effet, en ne reconstruisant que le ligament coraco-claviculaire trapézoïde, la liaison entre la clavicule et l'apophyse coracoïde est assurée d'une manière différente de la liaison naturellement réalisée par les deux ligaments coraco-claviculaire (le ligament coraco-claviculaire trapézoïde et le ligament coraco-claviculaire conoïde). Cette différence risque de provoquer des gênes ou des douleurs résiduelles pour le patient.

KR102035651 B1 divulgue un instrument chirurgical pour la reconstruction du ligament de l'épaule comprenant : un premier dispositif de forage forant la clavicule et la coracoïde ; un deuxième dispositif de forage forant la clavicule ; et un dispositif d'alignement de forage reliant le premier et le deuxième dispositif de forage de manière à être espacés radialement autour du point de forage de la coracoïde.

US 2012/253352 A2 divulgue un guide pour articulation acromioclaviculaire permettant de former plusieurs tunnels de forage espacés d'une distance prédéterminée et utilisant deux guides de forage disposés en pivot, permettant à un guide de forage de pivoter autour de l'axe de l'autre.

### Présentation de l'invention

La présente invention vise à remédier aux inconvénients précités en proposant un instrument ancillaire destiné à la chirurgie de la disjonction acromio-claviculaire.

A cet effet, l'instrument ancillaire selon l'invention permet une reconstruction des ligaments coraco-claviculaire trapézoïde et coraco-claviculaire conoïde, tout en protégeant les zones denses en nerfs et en artères de l'épaule du patient. L'instrument ancillaire de la présente invention comporte :
- Une poignée, en forme d'arc de cercle, présentant deux extrémités, dite première et seconde extrémité.
- Un bras, de forme longitudinale, fixé au niveau d'une première extrémité à la première extrémité de la poignée et équipé, au niveau d'une seconde extrémité, d'une pièce en forme de cuillère, dite cuillère.
- Un guide de perçage, lié à la seconde extrémité de la poignée, présentant :
   ∘ un orifice traversant dit central, dont un axe longitudinal passe par ladite cuillère,
   ∘ deux orifices traversants dits auxiliaires, disposés de manière diamétralement opposés par rapport à l'orifice central, chacun des deux orifices traversants auxiliaires ayant un axe longitudinal passant par ladite cuillère, les deux orifices traversants auxiliaires formant une paire d'orifices traversants auxiliaires.
- Un canon de perçage dit central, adapté pour s'insérer dans l'orifice traversant central.
- Deux canons de perçage dits auxiliaires adaptés pour s'insérer dans les deux orifices traversants auxiliaires.

La poignée permet de manipuler l'instrument ancillaire.

Le canon de perçage central présente une forme de tube creux dans lequel peut s'insérer un foret canulé, appelé également la suite foret.

On entend par un foret canulé, une mèche canulée ou un foret en forme de tube creux qui permet de percer des os.

Dans le cadre d'une opération sous arthroscopie visant à traiter cette disjonction acromio-claviculaire, un praticien pourra choisir entre une reconstruction du ligament coraco-claviculaire trapézoïde, dite reconstruction du tunnel coracoïdien, et une reconstruction des ligaments coraco-claviculaire trapézoïde et conoïde, dite opération de laçage. Cette opération de laçage présentant l'avantage de recréer une liaison entre une clavicule et une apophyse coracoïde plus proche de la liaison naturellement assurée par les ligaments entre ces deux os. Cette opération de laçage est donc plus anatomique que la reconstruction du tunnel coracoïdien.

La reconstruction du tunnel coracoïdien nécessite un unique perçage de la clavicule et de l'apophyse coracoïde selon un unique axe. L'opération de laçage nécessite deux perçages de la clavicule, chaque perçage selon respectivement deux axes passant sous l'apophyse coracoïde et ne traversant pas l'apophyse coracoïde.

Lors d'une reconstruction du tunnel coracoïdien, le praticien effectue préalablement deux incisions sur le corps d'un patient, une première incision au-dessus de la clavicule et une deuxième incision en dessous de l'apophyse coracoïde, aussi appelée coracoïde.

Le praticien insère le canon de perçage principal dans l'orifice traversant central du guide de perçage.

Puis, le praticien introduit le canon de perçage central par la première incision, jusqu'à être en appui sur la clavicule.

Le praticien insère ensuite la cuillère dans la deuxième incision, jusqu'à placer la cuillère en appui sous la coracoïde. Le praticien pourra ensuite percer à l'aide d'un foret canulé, guidé par le canon de perçage central, la clavicule puis la coracoïde jusqu'à atteindre la cuillère.

L'axe longitudinal de l'orifice central du guide de perçage, et donc du canon de perçage principal, permet au forêt canulé d'atteindre la cuillère après avoir percé la clavicule puis la coracoïde.

Lors d'une opération de laçage, de manière similaire à une reconstruction du canal coracoïdien, le praticien effectue deux incisions sur le corps du patient, une première au-dessus de la clavicule et une deuxième, soit sur une face avant de l'épaule en dessous de la coracoïde, soit sur face latéral de l'épaule juste au-dessus de l'humérus.

Le praticien insère le canon de perçage principal dans l'orifice traversant central du guide de perçage et chaque canon de perçage auxiliaire dans un orifice traversant auxiliaire.

Puis, le praticien introduit le canon de perçage central par la première incision, jusqu'à venir en appui sur la clavicule.

Le praticien insère ensuite la cuillère, équipée sur la seconde extrémité du bras, dans la deuxième incision, jusqu'à placer la cuillère en appui sous la coracoïde.

Le praticien pourra ensuite percer à l'aide d'un foret canulé, guidé par le canon de perçage central, la clavicule puis la surface de la coracoïde jusqu'à une première corticale. On entend par première corticale, une couche superficielle d'un os. L'instrument ancillaire est ainsi maintenu en position par rapport à la clavicule et à la coracoïde. Le praticien perce ensuite la clavicule, deux fois, avec deux forets canulés guidés chacun par un canon de perçage auxiliaire. Les deux forets canulés guidés respectivement par chaque canon de perçage auxiliaires traversent la clavicule, puis bordent de part et d'autre la coracoïde pour ensuite atteindre la cuillère.

L'instrument ancillaire selon l'invention permet ainsi, tout en limitant les risques encourus par le patient, d'effectuer les perçages nécessaires, pour soit une reconstruction du canal coracoïdien soit une opération de laçage.

En effet, les canons de perçage adaptés pour s'insérer dans les orifices traversant selon des axes longitudinaux passant par la cuillère du guide de perçage permettent de guider des forets canulés lors des perçages nécessaires à ces opérations tout en protégeant, grâce à la cuillère, la zone dense en nerfs et en artères du patient.

Dans des modes particuliers de réalisation l'invention peut comporter en outre l'une ou plusieurs des caractéristiques suivantes, prises isolément ou selon toutes les combinaisons techniquement possibles.

Selon un mode de réalisation, le guide de perçage comporte plusieurs paires d'orifices traversants auxiliaires et, pour chaque paire d'orifices traversants auxiliaires, les deux orifices traversants auxiliaires sont disposés de manière diamétralement opposés par rapport à l'orifice central, et ont chacun un axe longitudinal passant par ladite cuillère.

Lors d'une opération de laçage, le praticien pourra adapter l'opération aux variations de forme et d'emplacement de la coracoïde selon les patients. En effet, en disposant de plusieurs paire d'orifices traversants auxiliaires, il pourra choisir la paire d'orifices traversants auxiliaires adaptée au contournement de la coracoïde lors des deux perçages de la clavicule. C'est-à-dire, de manière à ce que les forets canulés ne percent pas la coracoïde avant d'atteindre la cuillère.

Selon un mode de réalisation, le bras présente un cintrage dont la courbure est orientée vers la poignée.

Le bras présentant un cintrage permet avantageusement d'utiliser une voie d'abord dite latérale. On entend par « voie d'abord », la zone du corps du patient sur laquelle on pratique les incisions au début de l'opération.

La reconstruction du tunnel coracoïdien et l'opération de laçage peuvent être réalisées selon une voie d'abord dite antérieure, c'est-à-dire que l'incision pratiquée pour insérer la cuillère et le bras est faite au niveau de l'épaule sur la face avant du corps du patient. Cependant, cette zone du corps du patient présente une artère importante, cette voie d'abord présente donc plus de risques pour le patient.

Lors de l'utilisation de la voie d'abord latérale, l'incision pratiquée pour insérer la cuillère et le bras est faite au niveau de l'épaule sur la face latérale du corps du patient. Cela concerne une zone du corps du patient ne présentant pas d'artères importantes. Cependant, en utilisant cette voie d'abord, l'humérus du bras du patient empêche un accès rectiligne à la coracoïde. Le bras présentant un cintrage dont la courbure est orientée vers la poignée permet de contourner l'humérus par le dessus pour atteindre la coracoïde et permet ainsi d'utiliser cette voie d'abord latérale comportant moins de risque pour le patient.

Selon un mode de réalisation, le canon de perçage central comporte, au niveau d'une extrémité, dite libre, orientée vers ladite cuillère, une rugine de Jacob.

On entend par rugine de Jacob, une pièce de forme allongée et recourbée comme une griffe, adaptée pour gratter ou racler un os.

Lors d'une reconstruction du tunnel coracoïdien ou lors d'une opération de laçage, le canon de perçage central est appuyé contre la clavicule au niveau de l'extrémité libre, et la rugine de Jacob permet de s'accrocher sur le rebord de la clavicule et d'ainsi assurer un meilleur maintien en position de l'instrument ancillaire lors des perçages.

Selon un mode de réalisation, la poignée est réglable. Les patients présentent des variations dans les dimensions et les emplacements de leur clavicule, de leur coracoïde et de leur humérus. La poignée réglable, en longueur notamment, permet d'adapter la distance entre le guide de perçage et le bras à ces différentes variations lors de l'utilisation de l'instrument ancillaire. La poignée est agencée de telle sorte qu'un centre de l'arc de cercle est situé au niveau de la cuillère , cela permet d'assurer que les axes longitudinaux des orifices traversants central et auxiliaires du guide de perçage passent toujours par la cuillère quel que soit le réglage de la poignée.

Selon un mode de réalisation, le bras présente un canal traversant, s'étendant entre la première extrémité du bras et la cuillère.

Le canal traversant permet avantageusement le passage d'une boucle de fil en Nitinol, dite boucle de Nitinol.

Lors d'une opération de laçage, avant le début de l'opération, une boucle de Nitinol est insérée dans le canal traversant du bras. Une extrémité de cette boucle de Nitinol est placée dans la cuillère, une autre extrémité, dite libre, est gardée à l'extérieur du canal traversant au niveau de la première extrémité du bras.

Après avoir réalisé les deux perçages, dits perçages auxiliaires, de la clavicule à l'aide des canons de perçage auxiliaires et après que les forets canulés ont atteint la cuillère, le praticien insère un fil chirurgical dans chacun des deux forets canulés placés dans les canons de perçage auxiliaires. Le fil chirurgical présente deux extrémités dite première et deuxième extrémités. Chaque fil chirurgical est inséré jusqu'à ce que leur deuxième extrémité dépasse de l'ouverture du foret canulé au niveau de la cuillère tout en maintenant leur première extrémité à l'extérieur du patient. Le praticien tire ensuite sur l'extrémité libre de la boucle de Nitinol. Lorsque la boucle de Nitinol est tirée, son extrémité placée dans la cuillère enserre la deuxième extrémité des fils chirurgicaux dépassant des forets canulés. Le praticien retire ensuite le bras et la cuillère du corps du patient, entrainant ainsi grâce à la boucle de Nitinol la deuxième extrémité des fils chirurgicaux à l'extérieur du corps du patient. A la suite de ces étapes, le praticien dispose de deux fils chirurgicaux dont chaque première extrémité est à l'extérieur du patient au niveau de la première incision et chaque deuxième extrémité à l'extérieur du patient au niveau de la deuxième incision. Chaque fil chirurgical traverse la clavicule du patient. Le praticien pourra ensuite utiliser ces fils chirurgicaux pour implanter un ligament artificiel constitué, par exemple, d'un premier fil reliant un implant dit bouton de clavicule destiné à être placé sur la clavicule au niveau d'un des deux perçages auxiliaires à un implant dit bouton de coracoïde destiné à être placé sous la coracoïde et d'un deuxième fil reliant un deuxième bouton de clavicule destiné à être placé sur la clavicule au niveau de l'autre perçage auxiliaire au bouton de coracoïde. En effet, les différents implants peuvent être plus facilement fixés aux fils par le praticien à l'extérieur du corps du patient.

Le bras présentant un canal traversant, s'étendant entre la première extrémité du bras et la cuillère permet donc avantageusement au praticien de plus facilement placer les constituants d'un ligament artificiel à l'aide d'une boucle de Nitinol et de fils chirurgicaux.

Selon un mode de réalisation, l'instrument ancillaire comporte également au moins un foret canulé destiné à être inséré dans un canon de perçage central ou auxiliaire.

Un foret canulé présente une forme de tube creux dans lequel pourra être inséré un ou plusieurs fils chirurgicaux ou encore une boucle de Nitinol. Le au moins un foret canulé inclus dans l'instrument ancillaire présente une forme et un diamètre adaptés au canon de perçage utilisé.

Selon un mode de réalisation, le au moins un foret canulé présente une rainure représentative d'une profondeur de perçage prédéterminée. La rainure peut être par exemple, une rainure circulaire réalisée sur une surface circonférentielle extérieure du foret canulé. La rainure peut également être une rainure longitudinale réalisée sur la surface longitudinale extérieure du foret canulé. Cette rainure longitudinale présente une longueur s'étendant entre deux extrémités. Lorsque l'on souhaite percer selon une profondeur prédéterminée, par exemple, dans le cadre d'une opération de laçage, le praticien pourra obtenir une indication que la première corticale de la coracoïde est atteinte lorsque, dans le cas d'une rainure circulaire, ladite rainure circulaire est visible juste au-dessus d'une extrémité du canon de perçage central, située du côté du guide de perçage,. Le praticien pourra également obtenir une indication que la première corticale de la coracoïde est atteinte lorsque, dans le cas d'une rainure longitudinale, une des extrémités de ladite rainure longitudinale est au niveau de l'extrémité du canon de perçage central située du côté du guide de perçage. Ce au moins un foret canulé présentant une rainure sera utilisé préférentiellement avec le canon de perçage principal.

Selon un mode de réalisation, le au moins un foret canulé présente une butée configurée pour prendre appui sur le canon de perçage utilisé. Un foret canulé présente en général un diamètre sensiblement constant le long du foret canulé. On entend par butée, une augmentation locale du diamètre extérieur du foret empêchant le foret d'être enfoncé au-delà de cette butée dans un canon de perçage. Cela permet d'empêcher le foret de réaliser un perçage plus profond qu'une profondeur prédéterminée.

Selon un mode de réalisation, Par exemple, la cuillère peut présenter des orifices traversants dans son épaisseur, destinés à accueillir les extrémités des forets canulés guidés par les canons de perçage auxiliaires. Ces orifices traversants de la cuillère pourront, dans le cadre d'une opération de laçage, faciliter la récupération, par la boucle de Nitinol dans la cuillère, de fils chirurgicaux insérés dans les forets canulés. Dans ce cas, la cuillère n'empêche plus les forets canulés d'atteindre la zone dense en nerfs et en artères du patient sous la coracoïde. Un foret canulé présentant une butée configurée pour prendre appui sur le canon de perçage utilisé empêchera le foret canulé d'atteindre cette zone dense en nerfs et en artères. Ce au moins un foret canulé présentant une butée sera utilisé préférentiellement avec les canons de perçage auxiliaires.

### Présentation des figures

L'invention sera mieux comprise à la lecture de la description suivante, donnée à titre d'exemple nullement limitatif, et faite en se référant aux figures :
[Fig. 1] une représentation schématique d'une reconstruction du tunnel coracoïdien,
[Fig. 2] une représentation schématique d'une opération de laçage,
[Fig. 3] une représentation schématique d'un exemple de réalisation de l'instrument ancillaire selon l'invention,
[Fig. 4] une représentation schématique d'exemples de réalisation de forets canulés,
[Fig. 5] une représentation schématique de l'exemple de réalisation de l'instrument ancillaire utilisé lors d'une opération de laçage,
[Fig. 6] une représentation schématique d'un exemple de réalisation d'implants utilisés dans une reconstruction du tunnel coracoïdien ou dans une opération de laçage,
[Fig. 7] une représentation schématique d'un exemple de réalisation d'un bras et d'une cuillère de l'instrument ancillaire,
[Fig. 8] une représentation schématique d'un exemple de réalisation d'une cuillère de l'instrument ancillaire.

Dans ces figures, des références identiques d'une figure à une autre désignent des éléments identiques ou analogues. Pour des raisons de clarté, les éléments représentés ne sont pas nécessairement à une même échelle, sauf mention contraire.

### Description détaillée des modes de réalisation particuliers de l'invention

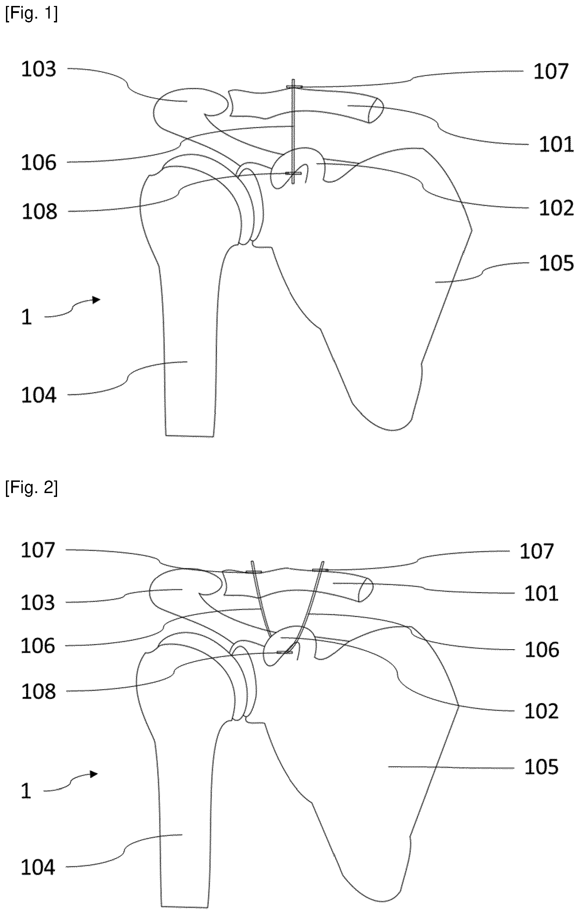

La figure 1 représente une épaule 1 d'un patient. Une clavicule 101, une apophyse coracoïde, appelée par la suite coracoïde 102, un acromion 103, un humérus 104 et une omoplate 105 sont également visibles. La figure 1 illustre schématiquement une reconstruction du ligament coraco-claviculaire trapézoïde, aussi appelée reconstruction du tunnel coracoïdien. La reconstruction du tunnel coracoïdien consiste à placer un ligament artificiel constitué d'un fil 106, tendu entre un implant, dit bouton de clavicule 107, placé sur la clavicule et un implant, dit bouton de coracoïde 108, placé sous la coracoïde. La reconstruction du tunnel coracoïdien nécessite le perçage de la clavicule 101 et de la coracoïde 102. Le perçage de la clavicule 101 et de la coracoïde 102 doit être réalisé selon un même axe afin de permettre au fil 106 d'être placé de manière adéquate.
La figure 2 illustre schématiquement une reconstruction des ligaments coraco-claviculaires trapézoïde et conoïde, appelée opération de laçage. L'opération de laçage nécessite deux perçages de la clavicule 101 afin de placer un ligament artificiel constitué de deux fils 106, mais aucun perçage de la coracoïde 102. Deux boutons de clavicule 107 sont placés sur la clavicule et un bouton de coracoïde 108 est placé sous la coracoïde. Un fil 106 est tendu entre chaque bouton de clavicule 107 et le bouton de coracoïde 108. Lors d'une opération de laçage, les deux fils 106 traversent la clavicule mais ne traversent pas la coracoïde. Les perçages de la clavicule 101 doivent être réalisés selon des axes passant sous la coracoïde afin de pouvoir placer les fils 106 de manière adéquate. La position des fils 106 de part et d'autre de la coracoïde permettent à l'opération de laçage d'être plus anatomique que la reconstruction du tunnel coracoïdien. On entend par « plus anatomique », le fait que les ligaments artificiels attachent les différents os entre eux d'une manière plus proche de la manière dont les ligaments naturels attachent ces mêmes os entre eux.

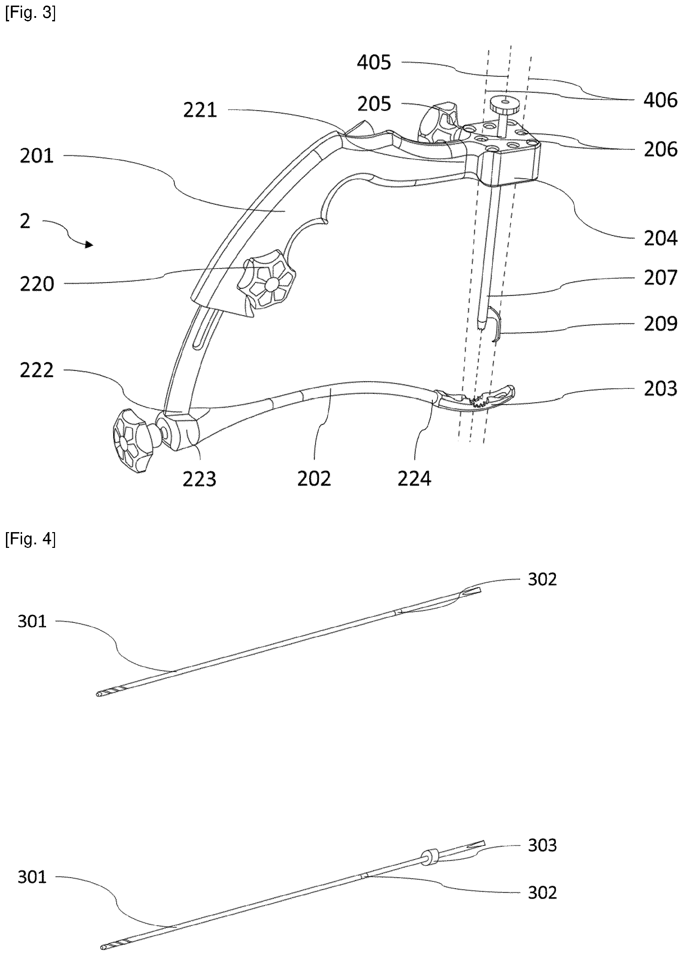

La figure 3 représente schématiquement un exemple de réalisation d'un instrument ancillaire 2 selon l'invention. L'instrument ancillaire 2 selon l'invention peut être avantageusement utilisé à la fois pour la reconstruction du tunnel coracoïdien et pour l'opération de laçage.

L'instrument ancillaire 2 comporte une poignée 201 en forme d'arc de cercle.

La poignée 201 présente deux extrémités, une première extrémité 222 à laquelle est fixé un bras 202 et une deuxième extrémité 221 à laquelle est fixé un guide de perçage 204.

Le bras 202 est de forme longitudinale. Il s'étend entre deux extrémités. Une première extrémité 223 du bras est fixée à la première extrémité 222 de la poignée. Une deuxième extrémité 224 du bras 202 est équipée d'une pièce appelée cuillère 203. La cuillère 203 est une pièce, par exemple en matériau métallique, dont la forme permet de s'adapter à la forme de la coracoïde 102 afin de s'y appuyer par en-dessous.

La poignée 201 est agencée de telle sorte qu'un centre de l'arc de cercle est situé au niveau de la cuillère.

Dans un exemple préféré de réalisation, illustré sur la figure 3, la poignée 201 est réglable. Ainsi, l'instrument ancillaire permet d'adapter la longueur entre le bras 202 et le guide de perçage 204 à différentes morphologies d'épaule 1 de patient.

Dans une forme de réalisation, la poignée 201 présente une fente dans laquelle coulisse un élément de guidage, également en arc de cercle. Un moyen de verrouillage 220 permet de maintenir en position l'élément de guidage dans la poignée.

Lors du réglage de la poignée 201, une fois que la longueur entre le bras 202 et le guide de perçage 204 est adaptée à la morphologie du patient, un praticien pourra verrouiller la poignée au moyen du moyen de verrouillage 220.

Dans une forme préférée de réalisation, comme illustré sur la figure 3, le bras 202 présente un cintrage dont la courbe est orientée vers la poignée 201. Une telle forme du bras 202 permet avantageusement de contourner l'humérus 104 pour atteindre la coracoïde avec la cuillère 203, lorsqu'une voie d'abord latérale est utilisée pour l'insertion de l'instrument ancillaire dans le corps du patient. On entend par voie d'abord latérale le fait qu'une incision, réalisée pour insérer la cuillère et le bras dans le corps du patient, est faite au niveau d'une face latérale de l'épaule 1 du patient.

Dans une autre forme de réalisation, le bras 202 ne présente pas de cintrage. Dans ce cas, une voie d'abord antérieure pourra être utilisée pour l'insertion de l'instrument ancillaire dans le corps du patient. On entend par voie d'abord antérieure le fait qu'une incision, réalisée pour insérer la cuillère et le bras dans le corps du patient, est faite depuis une face avant de l'épaule du patient. Cela permet d'atteindre la coracoïde 102 sans avoir à contourner l'humérus 104.

Le guide de perçage 204 présente un orifice traversant dit central 205 dont un axe longitudinal 405 passe par la cuillère 203. Un canon de perçage central 207 est adapté pour s'insérer dans l'orifice traversant central 205. Le canon de perçage central 207 a la forme d'un tube creux configuré pour accueillir et guider un foret canulé 301, aussi appelé foret. L'orifice traversant central 205 dont l'axe longitudinal 405 passe par la cuillère permet de garantir qu'un foret canulé guidé par le canon de perçage central débouche forcément dans la cuillère 203.

Le guide de perçage 204 comporte des orifices traversants dits auxiliaires 206. Chaque orifice traversant auxiliaire 206 est orienté selon un axe longitudinal 406 traversant la cuillère 203. Les orifices traversants auxiliaires 206 du guide de perçage 204 sont destinés à accueillir des canons de perçage auxiliaires 208. Des forets canulés 301 sont destinés à être insérés dans les canons de perçage auxiliaires 208. Les orifices traversants auxiliaires 206 et les canons de perçages auxiliaires sont avantageusement destinés pour les opérations de laçage, comme il sera décrit ultérieurement. Seuls deux orifices traversants auxiliaires 206 et deux canons de perçages auxiliaires sont utilisés pour une opération de laçage,

Les orifices traversants auxiliaires 206 du guide de perçage 204 sont disposés par paires diamétralement opposées autour de l'orifice traversant central 205.

Dans un mode de réalisation (non représenté), le guide de perçage 204 présente une seule paire d'orifices traversants auxiliaires 206. Les deux orifices traversants auxiliaires de la paire d'orifices traversants auxiliaires et l'orifice traversant central sont alignés.

Dans un autre mode de réalisation, le guide de perçage 204 présente plusieurs paires d'orifices traversants auxiliaires. Cela permet au praticien dans le cadre d'une opération de laçage de s'adapter à la morphologie du patient en sélectionnant la paire d'orifices traversant auxiliaires permettant de ne pas percer la coracoïde.

Dans l'exemple préféré mais non limitatif de la figure 3, le guide de perçage comporte huit orifices traversants auxiliaires.

Dans un mode de réalisation préféré, comme illustré sur la figure 3, le canon de perçage central 207 comporte, au niveau d'une l'extrémité libre, orientée vers la cuillère 203, une rugine de Jacob 209. La rugine de Jacob est une pièce, généralement en matériau métallique, d'une forme allongée et recourbée sensiblement similaire à la forme d'une griffe. Cette rugine de Jacob 209 permet avantageusement d'augmenter la stabilité de l'instrument ancillaire en s'accrochant à un rebord de la clavicule 101 avant la réalisation du perçage.

La figure 4 représente schématiquement deux exemples de réalisation de forets canulés 301. Un foret canulé 301, aussi appelé mèche canulée, est une pièce allongée en forme de tube creux adapté pour être implanté dans le corps d'un patient et pour percer des os. Le foret canulé 301 est creux de manière à pouvoir accueillir des fils chirurgicaux ou une boucle en Nitinol.

Dans un exemple de réalisation, le foret canulé comporte une rainure 302, sur une surface circonférentielle dudit foret canulé 301, permettant d'indiquer au praticien que le foret canulé a atteint une profondeur recherchée. Le praticien pourra, par exemple, considérer que la profondeur recherchée est atteinte lorsque la rainure 302 se trouve juste au-dessus d'une extrémité du canon de perçage central, située du côté du guide de perçage 204.

Dans un exemple de réalisation, le foret canulé comporte une butée 303 présente sur une surface circonférentielle dudit foret canulé 301. Cette butée se présente sous la forme d'une augmentation localisée du diamètre extérieur du foret canulé. Cette butée est configurée pour prendre appui sur le canon de perçage utilisé, permettant ainsi avantageusement d'empêcher le foret canulé 301 de dépasser une profondeur prédéterminée. Des zones sensibles du corps du patient sont ainsi protégées lors de la réalisation d'un perçage.

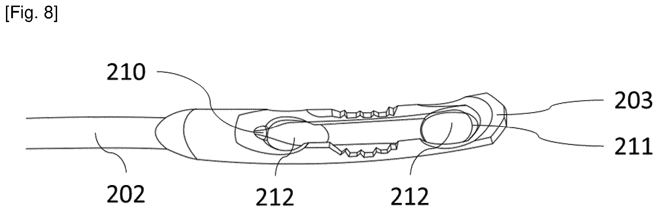

Dans un mode de réalisation, comme illustré sur la figure 8, la cuillère présente deux ouvertures 212 traversantes dans son épaisseur. Chacune des deux ouvertures est située au niveau d'une intersection virtuelle d'un axe longitudinal d'un orifice traversant auxiliaire du guide de perçage avec la cuillère. Les forets guidés par les canons de perçage auxiliaires traversent les deux ouvertures de la cuillère. Dans ce cas, l'utilisation de forets comportant une butée est particulièrement avantageuse pour protéger la zone sensible sous la coracoïde.

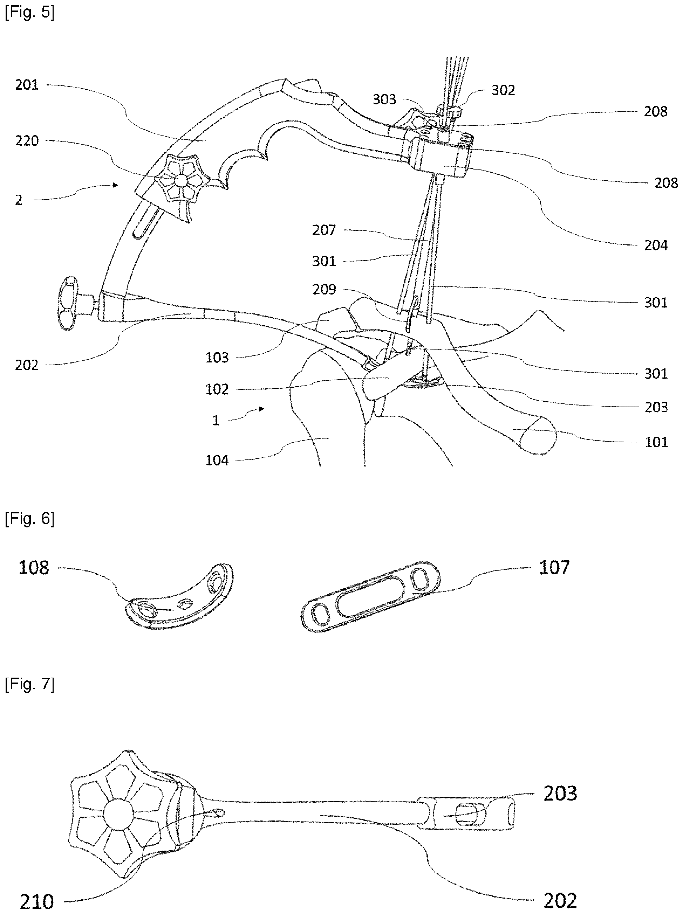

Dans un mode de réalisation, comme illustré sur la figure 7, le bras 202 présentant un canal traversant 210, s'étendant entre la première extrémité 223 du bras et la cuillère 203. Le canal traversant 210 est avantageusement utilisé pour les opérations de laçage, comme il sera décrit ultérieurement, notamment pour le passage de fil chirurgicaux ou d'une boucle en fil de Nitinol. Par exemple, et comme illustré sur la figure 8, une première extrémité d'une boucle 211 de fil en Nitinol, dite boucle de Nitinol, dépasse du canal traversant 210 de manière à ce que la première extrémité de la boucle de Nitinol soit placée dans la cuillère 203.

La figure 6 est une représentation schématique d'implants utilisés dans une opération de reconstruction du tunnel coracoïdien ou dans une opération de laçage. On entend par implant, une pièce destinée à rester à demeure dans le corps d'un patient après une intervention chirurgicale. Un premier implant 108, dit bouton de coracoïde, est représenté sur cette figure 6. le bouton de coracoïde est de forme courbée permettant avantageusement de s'adapter à la forme de la coracoïde pour être placé en appui en dessous de la coracoïde. Un deuxième implant 107, dit bouton de clavicule, est également représenté sur la figure 6. Ce bouton de clavicule 107 est destiné à être placé sur la clavicule 101 lors d'une opération de laçage ou lors d'une reconstruction du canal coracoïdien.

Dans un exemple de mise en œuvre de l'instrument ancillaire 2 dans le cadre d'une reconstruction du tunnel coracoïdien, le praticien a recours uniquement au canon de perçage central. Les canons de perçages auxiliaires ne sont pas utilisés. Le bras 202 de l'instrument ancillaire est cintré afin d'utiliser une voie d'abord latérale et contourner l'humérus. Le praticien réalise deux incisions sur l'épaule du patient. Une première incision au-dessus de la clavicule 101 et une deuxième incision au niveau de l'interstice entre l'humérus 104 et l'acromion 103. Il manipule ensuite l'instrument ancillaire 2 à l'aide de la poignée 201 de la manière suivante :
- il insère l'extrémité libre du canon de perçage central 207 dans la première incision jusqu'à ce que l'extrémité libre du canon de perçage central 207 soit en appui sur la clavicule 101.
- il insère ensuite la cuillère 203, équipée sur une extrémité du bras 202, jusqu'à placer la cuillère 203 en appui sous la coracoïde.

Lorsque le canon de perçage principal comporte un rugine de Jacob, la rugine de Jacob vient s'accrocher à la clavicule, comme illustré sur la figure 5.

Le praticien pourra ensuite réaliser le perçage nécessaire à la reconstruction du tunnel coracoïdien à l'aide d'un foret canulé 301 guidé par le canon de perçage central 207. Le foret canulé 301 est utilisé pour percer la clavicule 101 puis la coracoïde 102. La zone du corps du patient sous la coracoïde 102 est une zone, dite sensible, riche en nerfs et en artères. Lors du perçage, la cuillère 103 empêche avantageusement le foret canulé 301 d'atteindre cette zone sensible en bloquant une pointe du foret canulé juste après que la coracoïde soit percée.

Dans un exemple de mise en œuvre de l'instrument ancillaire 2 dans le cadre d'une opération de laçage, comme illustré sur la figure 5, le praticien a recours au canon de perçage central et à deux canons de perçage auxiliaires. Le bras 202 de l'instrument ancillaire est cintré afin d'utiliser une voie d'abord latérale et contourner l'humérus. Le praticien réalise également deux incisions sur l'épaule du patient. Une première incision au-dessus de la clavicule 101 et une deuxième incision au niveau de l'interstice entre l'humérus 104 et l'acromion 103. Il manipule ensuite l'instrument ancillaire 2 à l'aide de la poignée 201 de la manière suivante :
- il insère l'extrémité libre du canon de perçage central 207 dans la première incision jusqu'à ce que l'extrémité libre du canon de perçage central 207 soit en appui sur la clavicule 101.
- il insère ensuite la cuillère 203 jusqu'à placer la cuillère 203 en appui sous la coracoïde.
Lorsque le canon de perçage principal comporte une rugine de Jacob, la rugine de Jacob vient s'accrocher à la clavicule, comme illustré sur la figure 5.

Un foret canulé est ensuite inséré dans le canon de perçage central 207 de manière à percer la clavicule 101 et à atteindre la première corticale de la coracoïde 102. On entend par première corticale, la couche superficielle d'un os. Ce perçage de la clavicule guidé par le canon de perçage central n'est généralement pas utilisé pour insérer un ligament artificiel dans le cadre d'une opération de laçage. Il permet cependant d'assurer un maintien en position de l'instrument ancillaire 2 lors des perçages dits auxiliaires guidés par les canons de perçage auxiliaires.

Deux forets canulés 301 sont insérés dans deux canons de perçage auxiliaires 208 et permettent de percer en deux endroits la clavicule 101. Les canons de perçage auxiliaires ont été préalablement placés dans deux orifices traversants auxiliaires diamétralement opposés par rapport à l'orifice traversant central, selon la morphologie du patient, afin de ne pas percer la coracoïde avec les forets canulés.
Tel que représenté de manière non limitative sur la figure 5, le foret canulé inséré dans le canon de perçage central 207 comporte une rainure 302. Cette rainure permet avantageusement d'indiquer au praticien que la première corticale de la coracoïde est atteinte. Tels que représentés de manière non limitative sur la figure 5, les forets canulés insérés dans les canons de perçage auxiliaires comportent une butée 303. Cette butée 303 permet avantageusement de protéger la zone sensible sous la coracoïde.

La boucle de Nitinol 211 est insérée dans le canal traversant 210 de manière à ce que la première extrémité de la boucle de Nitinol soit placée dans la cuillère 203 et de manière à ce qu'une autre extrémité de la boucle de Nitinol, dite extrémité libre, dépasse du canal traversant au niveau de la première extrémité du bras 202. Après avoir réalisé les perçages auxiliaires, le praticien insère un fil chirurgical dans chacun des forets canulés placés dans les canons de perçage auxiliaires. Chaque fil chirurgical présente deux extrémités, dites première et deuxième extrémités. Chaque première extrémité du fil chirurgical est maintenue à l'extérieur du corps du patient au-dessus de la clavicule. Chaque deuxième extrémité du fil chirurgical dépasse du foret canulé à l'intérieur du corps du patient au niveau de la cuillère. Le praticien tire ensuite sur l'extrémité libre de la boucle de Nitinol. Lorsque la boucle de Nitinol est tirée, sa première extrémité s'accroche à la deuxième extrémité des fils chirurgicaux. Le praticien retire ensuite la cuillère du corps du patient. Lorsque la cuillère est retirée du corps du patient, la boucle de Nitinol entraine la deuxième extrémité de chaque fil chirurgical à l'extérieur du corps du patient. Le praticien dispose ainsi de deux fils chirurgicaux traversant la clavicule 101 et dont les extrémités sont à l'extérieur du corps du patients. Il pourra ensuite utiliser ces fils pour placer les boutons de clavicule 107 sur la clavicule au niveau de chaque perçage auxiliaire et pour placer le bouton de coracoïde 108 en appui sous la coracoïde à l'emplacement précédemment occupé par la cuillère 203. Grâce au canal traversant 210 du bras 202, le praticien peut facilement extraire les extrémités de fils chirurgicaux traversant la clavicule d'un patient, et peut ainsi plus facilement fixer des implants à ces fils chirurgicaux.

## Revendications

1. Instrument ancillaire (2) pour chirurgie de la disjonction acromio-claviculaire comportant :
- Une poignée (201), en forme d'arc de cercle, présentant deux extrémités, dite première (222) et seconde extrémité (221),
- Un bras (202), de forme longitudinale, fixé au niveau d'une première extrémité (223) à la première extrémité (222) de la poignée (201) et équipé, au niveau d'une seconde extrémité (224), d'une pièce en forme de cuillère, dite cuillère (203),
- Un guide de perçage (204), lié à la seconde extrémité (221) de la poignée (201), présentant :
∘ un orifice traversant dit central (205), dont un axe longitudinal (405) passe par ladite cuillère (203),
∘ deux orifices traversants dits auxiliaires (206), disposés de manière diamétralement opposés par rapport à l'orifice central (205), chacun des deux orifices traversants auxiliaires (206) ayant un axe longitudinal (406) passant par ladite cuillère (203), les deux orifices traversants auxiliaires (206) formant une paire d'orifices traversants auxiliaires,
- Un canon de perçage dit central (207) adapté pour s'insérer dans l'orifice traversant central (205),
- Deux canons de perçage dits auxiliaires (208) adaptés pour s'insérer dans les deux orifices traversants auxiliaires (206).

2. Instrument ancillaire (2) selon la revendication 1 dans lequel le guide de perçage (204) comporte plusieurs paires d'orifices traversants auxiliaires (206), et dans lequel, pour chaque paire d'orifices traversants auxiliaires, les deux orifices traversants auxiliaires (206) sont disposés de manière diamétralement opposés par rapport à l'orifice central (205), et ont chacun un axe longitudinal (406) passant par ladite cuillère (203).

3. Instrument ancillaire (2) selon l'une des revendications précédentes dans lequel le bras (202) présente un cintrage dont la courbure est orientée vers la poignée (201).

4. Instrument ancillaire (2) selon l'une des revendications précédentes dans lequel le canon de perçage central (207) comporte, au niveau d'une extrémité, dite libre, orientée vers ladite cuillère (203), une rugine de Jacob (209).

5. Instrument ancillaire (2) selon l'une des revendications précédentes dans lequel la poignée (201) est réglable.

6. Instrument ancillaire (2) selon l'une des revendications précédentes dans lequel le bras (202) présente un canal traversant (210), s'étendant entre la première extrémité du bras et la cuillère (203).

7. Instrument ancillaire (2) selon l'une des revendications précédentes comportant également au moins un foret canulé (301) destiné à être inséré dans un canon de perçage central (207) ou auxiliaire (208).

8. Instrument ancillaire (2) selon la revendication 7 dans lequel le au moins un foret canulé (301) présente une rainure (302) représentative d'une profondeur de perçage prédéterminée.

9. Instrument ancillaire (2) selon les revendications 7 ou 8 dans lequel le au moins un foret canulé (301) présente une butée (303) configurée pour prendre appui sur le canon de perçage utilisé.

## Patentansprüche

1. Hilfsinstrument (2) für Akromioklavikulardisjunktion-Chirurgie, aufweisend:
- einen bogenförmigen Griff (201), der zwei Enden aufzeigt, die als das erste Ende (222) und das zweite Ende (221) bezeichnet werden,
- einen länglichen Arm (202), der auf Höhe eines erstens Endes (223) an dem ersten Ende (222) des Griffs (201) befestigt ist und auf Höhe eines zweiten Endes (224) mit einem löffelförmigen Teil, der als Löffel (203) bezeichnet wird, ausgestattet ist,
- eine Bohrführung (204), die mit dem zweiten Ende (221) des Griffs (201) verbunden ist und Folgendes aufzeigt:
∘ eine mittlere Durchgangsöffnung (205), deren Längsachse (405) durch den Löffel (203) verläuft,
∘ zwei Hilfsdurchgangsöffnungen (206), die diametral entgegengesetzt in Bezug auf die mittlere Öffnung (205) angeordnet sind, wobei jede der zwei Hilfsdurchgangsöffnungen (206) eine Längsachse (406) aufweist, die durch den Löffel (203) verläuft, und die zwei Hilfsdurchgangsöffnungen (206) ein Paar von Hilfsdurchgangsöffnungen bilden,
- eine mittlere Bohrbüchse (207), die angepasst ist, um in die mittlere Durchgangsöffnung (205) eingefügt zu werden,
- zwei Hilfsbohrbüchsen (208), die angepasst ist, um in die zwei Hilfsdurchgangsöffnungen (206) eingefügt zu werden.

2. Hilfsinstrument (2) nach Anspruch 1, wobei die Bohrführung (204) mehrere Paare von Hilfsdurchgangsöffnungen (206) aufweist, und wobei bei jedem Paar von Hilfsdurchgangsöffnungen die zwei Hilfsdurchgangsöffnungen (206) diametral gegenüber in Bezug auf die mittlere Öffnung (205) angeordnet sind, und jeweils eine Längsachse (406) aufweisen, die durch den Löffel (203) verläuft.

3. Hilfsinstrument (2) nach einem der vorstehenden Ansprüche, wobei der Arm (202) eine Biegung aufzeigt, deren Krümmung zu dem Griff (201) hin ausgerichtet ist.

4. Hilfsinstrument (2) nach einem der vorstehenden Ansprüche, wobei die mittlere Bohrbüchse (207) auf Höhe eines freien Endes, das zu dem Löffel (203) ausgerichtet ist, eine Jacob-Riffelung (209) aufweist.

5. Hilfsinstrument (2) nach einem der vorstehenden Ansprüche, wobei der Griff (201) verstellbar ist.

6. Hilfsinstrument (2) nach einem der vorstehenden Ansprüche, wobei der Arm (202) einen Durchgangskanal (210) aufzeigt, der sich zwischen dem ersten Ende des Arms und dem Löffel (203) erstreckt.

7. Hilfsinstrument (2) nach einem der vorstehenden Ansprüche, auch aufweisend mindestens einen kanülierten Bohrer (301), der dazu bestimmt ist, in eine mittlere- (207) oder eine Hilfsbohrbüchse (208) eingeführt zu werden.

8. Hilfsinstrument (2) nach Anspruch 7, wobei der mindestens eine kanülierte Bohrer (301) eine Nut (302) aufzeigt, die repräsentativ für eine vorbestimmte Bohrtiefe ist.

9. Hilfsinstrument (2) nach den Ansprüchen 7 oder 8, wobei der mindestens eine kanülierte Bohrer (301) einen Anschlag (303) aufzeigt, der konfiguriert ist, um an der verwendeten Bohrbüchse anzuliegen.

## Claims

1. Ancillary instrument (2) for acromioclavicular disjunction surgery, having:
- A handle (201), in the shape of an arc of a circle, having two ends, called first (222) and second (221) ends,
- An arm (202), of longitudinal shape, fixed at a first end (223) to the first end (222) of the handle (201) and equipped, at a second end (224), with a spoon-shaped part, called a spoon (203),
- A drilling guide (204), connected to the second end (221) of the handle (201), having:
∘ a so-called central through-hole (205), a longitudinal axis (405) of which passes through said spoon (203),
∘ two so-called auxiliary through-holes (206), disposed diametrically opposite the central hole (205), each of the two auxiliary through-holes (206) having a longitudinal axis (406) passing through said spoon (203), the two auxiliary through-holes (206) forming a pair of auxiliary through-holes,
- A so-called central drilling bushing (207) suitable for being inserted into the central through-hole (205),
- Two so-called auxiliary drilling bushings (208) suitable for being inserted into the two auxiliary through-holes (206).

2. Ancillary instrument (2) according to claim 1, wherein the drilling guide (204) has a plurality of pairs of ancillary through-holes (206), and wherein, for each pair of ancillary through-holes, the two ancillary through-holes (206) are disposed diametrically opposite central hole (205), and each have a longitudinal axis (406) passing through said spoon (203).

3. Ancillary instrument (2) according to any one of the preceding claims, wherein the arm (202) has a bend, the curvature of which is oriented towards the handle (201).

4. Ancillary instrument (2) according to any one of the preceding claims, wherein the central drilling bushing (207) has, at a so-called free end, oriented towards said spoon (203), a Jacob's rugine (209).

5. Ancillary instrument (2) according to any one of the preceding claims, wherein the handle (201) is adjustable.

6. Ancillary instrument (2) according to any one of the preceding claims, wherein the arm (202) has a through-channel (210), extending between the first end of the arm and the spoon (203).

7. Ancillary instrument (2) according to any one of the preceding claims, also having at least one cannulated drill (301), intended to be inserted into a central (207) or auxiliary (208) drilling bushing.

8. Ancillary instrument (2) according to claim 7, wherein the at least one cannulated drill (301) has a groove (302) representative of a predetermined drilling depth.

9. Ancillary instrument (2) according to claims 7 or 8, wherein the at least one cannulated drill bit (301) has a stop (303) configured to bear on the drilling bushing used.
